# EUROPÄISCHE PATENTANMELDUNG

(11) **EP 1 442 714 A1**
(43) Veröffentlichungstag der Anmeldung: **04.08.2004**
(21) Anmeldenummer: 03002321.2
(22) Anmeldetag: 03.02.2003
(51) Int. Cl.: A61B 17/17

(54) **Zielhilfe für Rückenwirbel**

(71) Anmelder: Centerpulse Orthopedics Ltd., 6340 Baar (CH)
(72) Erfinder: Casutt, Guido, 8544 Rickenbach-Sulz (CH); Heller, Mathias, 8352 Räterschen (CH); Frei, Diego, 8302 Kloten (CH)
(74) Vertreter: Manitz, Finsterwald & Partner GbR

(57) **Zusammenfassung**

Die Erfindung betrifft eine Zielhilfe zur räumlichen Festlegung einer Zielvorrichtung relativ zu Rückenwirbeln eines Patienten, mit einem als Zange ausgebildeten Klemmwerkzeug (13), das wenigstens zwei Klemmbacken (15) aufweist, mit denen das Klemmwerkzeug wenigstens zwei benachbarte Rückenwirbel überbrückend an den Rückenwirbeln festklemmbar ist, und wenigstens einer fest mit dem Klemmwerkzeug verbundenen Zielbasis (11), die zur Koppelung mit einer separaten Zielvorrichtung ausgebildet ist.

## Beschreibung

Die Erfindung betrifft eine Zielhilfe zur räumlichen Festlegung einer Zielbasis relativ zu Rückenwirbeln eines Patienten.

Insbesondere bei Operationen an der Wirbelsäule besteht das Problem, dass der Operateur Implantate oder Befestigungsmittel wie beispielsweise Pedikelschrauben exakt positionieren bzw. ansetzen muss, um ein einwandfreies Operationsergebnis zu erzielen. Dies ist schwierig, da der Operateur in der Regel die korrekte Position bzw. Richtung nicht durch bloßes Sehen oder Tasten oder durch mehrmaliges Probieren ermitteln kann, sondern hierzu auf Hilfsmittel in Form spezieller Zielvorrichtungen angewiesen ist. Derartige Zielvorrichtungen sind beispielsweise in Form von Röntgeneinrichtungen bekannt, denen mittels eines festsetzbaren Schwenkarmes ein Zielgerät mit zwei entlang der optischen Achse beabstandeten Fadenkreuzen vorgesetzt werden kann, die bei korrekter Ausrichtung fluchten und im korrekt ausgerichteten Zustand gegen eine Führungsbüchse ausgetauscht werden, durch welche bei korrekter Ausrichtung des Zielgerätes ein Werkzeug hindurch geführt werden kann, beispielsweise eine Ahle, mit der an dem jeweiligen Rückenwirbel ein Ansatzpunkt markiert sowie ein Kanal für eine Pedikelschraube gebohrt wird.

Ein weiteres Problem besteht bei Navigationssystemen für die unverrückbare Befestigung eines Referenzkoordinatensystems an einem zu operierenden Knochen. Wenn es sich um einen einzelnen Rückenwirbel handelt, sind solche Referenzkoordinatensysteme, die beispielsweise einen Trägerkörper mit drei reflektierenden Kugeln beinhalten, sehr empfindlich in ihrer Befestigung.

Problematisch bei diesen und anderen Zielvorrichtungen sind die Schwierigkeiten bei deren korrekter Ausrichtung relativ zur Wirbelsäule bzw. relativ zu den Rückenwirbeln des Patienten. Hier will die Erfindung Abhilfe schaffen.

Aufgabe der Erfindung ist es daher, eine Möglichkeit zu schaffen, eine Zielvorrichtung auf einfache und zuverlässige Weise für den jeweiligen Operationszweck korrekt positionieren zu können.

Die Lösung dieser Aufgabe erfolgt durch die Merkmale des Anspruchs 1 und insbesondere dadurch, dass die Zielhilfe ein als Zange ausgebildetes Klemmwerkzeug, das wenigstens zwei Klemmbacken aufweist, mit denen das Klemmwerkzeug wenigstens zwei benachbarte Rückenwirbel überbrückend an den Rückenwirbeln festklemmbar ist, und wenigstens eine fest mit dem Klemmwerkzeug verbundene Zielbasis umfasst, die zur Koppelung mit einer separaten Zielvorrichtung ausgebildet ist.

Durch die Erfindung wird eine Klemmzange geschaffen, mit der auf denkbar einfache und dennoch äußerst zuverlässige Weise eine Zielbasis relativ zu den Rückenwirbeln räumlich fixiert werden kann. Hierdurch wird mit der Zielbasis ein Bezugspunkt geschaffen, dessen Lage relativ zu den mittels der Klemmbacken überbrückten Rückenwirbeln unveränderlich festgelegt ist, solange die Klemmzange an den Rückenwirbeln festgeklemmt ist. Mittels der auf diese Weise räumlich festgelegten Zielbasis kann nun eine separate Zielvorrichtung gekoppelt werden, die in eindeutig definierter und insbesondere reproduzierbarer Weise relativ zu den mittels der Klemmzange überbrückten Rückenwirbeln positioniert bzw. ausgerichtet werden kann.

Bevorzugte Ausführungsformen der Erfindung sind in den Unteransprüchen, der Beschreibung sowie der Zeichnung angegeben.

Die Klemmzange kann zwei um eine Drehachse drehbar miteinander verbundene Hebel aufweisen, die jeweils auf der einen Seite der Drehachse mit einem Betätigungsabschnitt versehen sind und auf der anderen Seite der Drehachse eine der Klemmbacken tragen.

Ferner ist bevorzugt vorgesehen, dass die Hebel zur Sicherung eines hergestellten Klemmzustandes relativ zueinander festsetzbar sind. Das Festsetzen, Fixieren oder Arretieren der Klemmzange kann dabei derart erfolgen, wie es von herkömmlichen Operationswerkzeugen her bekannt ist.

Die Hebel können derart ausgebildet sein, dass im festgeklemmten Zustand die Hebel zumindest im Bereich der Klemmbacken im Wesentlichen parallel zueinander verlaufen.

Des Weiteren kann vorgesehen sein, dass die Klemmbacken sich im Wesentlichen senkrecht zu einer von den beiden Hebeln aufgespannten Ebene erstrecken.

In einer weiteren bevorzugten Ausgestaltung der erfindungsgemäßen Zielhilfe ist die eine Klemmbacke fest und die andere Klemmbacke drehbar an dem jeweiligen Hebel angebracht. Hierdurch wird in vorteilhafter Weise ein weiterer Bewegungsfreiheitsgrad geschaffen, der zu einer verbesserten Ausrichtbarkeit der Klemmbacken relativ zu den zu überbrückenden Rückenwirbeln führt.

Ferner kann vorgesehen sein, dass zumindest eine Klemmbacke einen sich im Wesentlichen senkrecht zu einer von den beiden Hebeln aufgespannten Ebene erstreckenden Querträger umfasst, der wenigstens zwei mit Abstand voneinander angeordnete Klemmorgane trägt, die jeweils eine der anderen Klemmbacke zugewandte Klemmfläche aufweisen.

Bei entsprechend gewähltem Abstand der Klemmorgane kann hierdurch die erfindungsgemäße Klemmzange gezielt mit den Klemmorganen an den zu überbrückenden Rückenwirbeln bzw. an den Dornfortsätzen der Wirbel angesetzt werden.

Wenn gemäß einer bevorzugten Ausgestaltung beide Klemmbacken einen jeweils mit einem oder mehreren Klemmorganen versehenen Querträger umfassen, dann können die Klemmorgane in vorteilhafter Weise einander gegenüberliegend angeordnet sein.

Die Klemmflächen sind beispielsweise jeweils kreisförmig ausgebildet. Ferner können die Klemmflächen jeweils aufgeraut oder strukturiert sein. Eine Möglichkeit für eine Strukturierung besteht beispielsweise darin, in den Klemmflächen jeweils eine Mehrzahl von Kerben auszubilden.

Des Weiteren kann erfindungsgemäß vorgesehen sein, dass die Klemmorgane am Querträger beweglich gelagert sind. Hierdurch können sich die Klemmbacken besser an die zu überbrückenden Rückenwirbel anpassen.

Zwischen den Klemmorganen und dem Querträger kann jeweils eine Gelenkverbindung vorgesehen sein. Dabei kann es sich insbesondere um eine Kugelgelenkverbindung handeln.

Der Mittenabstand zwischen zwei benachbarten Klemmorganen einer Klemmbacke entspricht vorzugsweise dem Mittenabstand der Dornfortsätze zweier benachbarter Rückenwirbel im Lendenbereich.

Insbesondere kann der Mittenabstand zwischen zwei benachbarten Klemmorganen einer Klemmbacke im Bereich von etwa 32 mm ± 5 mm liegen.

Ein Vorteil dieser Anordnung besteht darin, dass sich jeweils ein starrer Teil der Klemmzange - nämlich jeweils die Klemmbacke - mit den schwenkbaren, insbesondere plattenförmig ausgebildeten Klemmorganen an den Dornfortsätzen in eindeutiger Weise ausrichtet, wenn die einander gegenüber liegenden, über die Klemmzange in vorgesehener Weise geführten Klemmorgane von der Gegenseite angepresst sind. Auf diese Weise werden die Wirbel über eine starre Brücke miteinander verbunden.

Die Zielbasis kann sich etwa parallel zu den Klemmbacken erstreckend von der Klemmzange abstehen. Insbesondere steht die Zielbasis etwa senkrecht von einem Hebel der Klemmzange ab.

Die Erfindung betrifft außerdem ein Zielsystem mit einer Zielhilfe, wie sie vorstehend erläutert wurde, und mit wenigstens einer separaten Zielvorrichtung, die mit der Zielbasis der Zielhilfe koppelbar ist.

Die Erfindung wird im Folgenden beispielhaft unter Bezugnahme auf die Zeichnung beschrieben. Es zeigen:
- Fig. 1: eine perspektivische Ansicht einer Zielhilfe gemäß einer Ausführungsform der Erfindung,
- Fig. 2: die Zielhilfe von Fig. 1 in einer Seitenansicht,

- Fig. 3: die Zielhilfe von Fig. 1 in einer Draufsicht mit in unterschiedlichen Stellungen befindlichen Klemmorganen,
- Fig. 4 und 5: jeweils ein im Schnitt dargestelltes, vergrößertes Detail der erfindungsgemäßen Zielhilfe, und
- Fig. 6: eine perspektivische Ansicht eines einen Bestandteil einer Klemmbacke bildenden Querträgers der erfindungsgemäßen Zielhilfe.

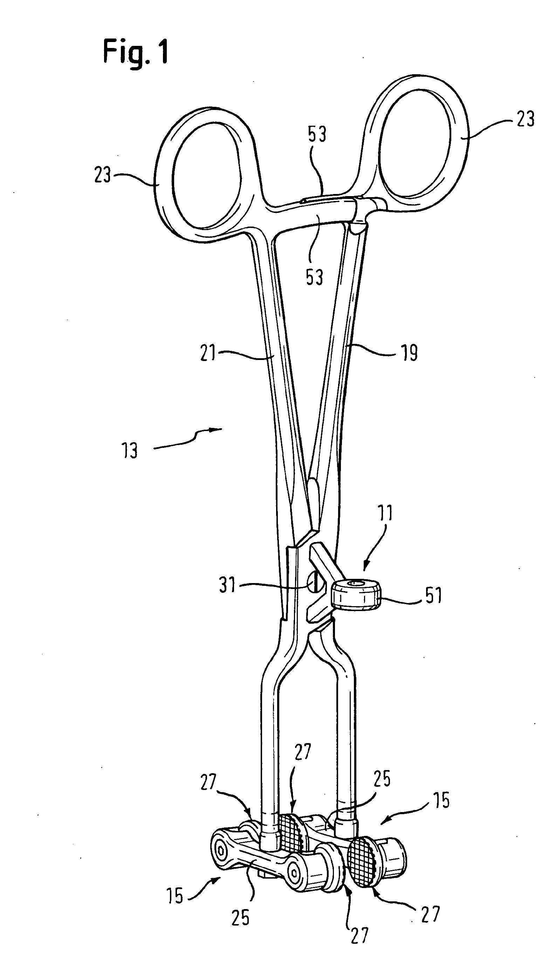

Die in den Fig. 1 bis 6 dargestellte erfindungsgemäße Zielhilfe umfasst eine Klemmzange 13 mit zwei um eine Drehachse 17 gelenkig miteinander verbundenen Hebeln 19, 21. Die Verbindung zwischen den beiden Hebeln 19, 21 im Bereich der Drehachse 17 erfolgt durch eine Gelenkschraube 31, die einen tragenden, zylindrisch ausgebildeten Abschnitt für den Hebel 19 aufweist und mit dem Hebel 21 verschraubt ist. Außerdem ist eine Drehsicherung zwischen der Gelenkschraube 31 und dem Hebel 21 vorgesehen.

An Betätigungsabschnitten 23 der Hebel 19, 21 gegenüber liegenden freien Hebelenden ist jeweils eine Klemmbacke 15 mit einem knochenförmigen, geraden Querträger 25 vorgesehen, der an seinen freien, senkrecht zu seiner Längserstreckung erweiterten Endbereichen jeweils ein beweglich gelagertes Klemmorgan 27 trägt, auf das nachstehend näher eingegangen wird. Dabei sind die Klemmorgane 27 der Hebel 19, 21 bei geschlossener Klemmzange 13 einander gegenüberliegend angeordnet, so dass die Klemmflächen 29 (auf die ebenfalls nachstehend näher eingegangen wird) der gegenüber liegenden Klemmorgane 27 direkt einander zugewandt sind.

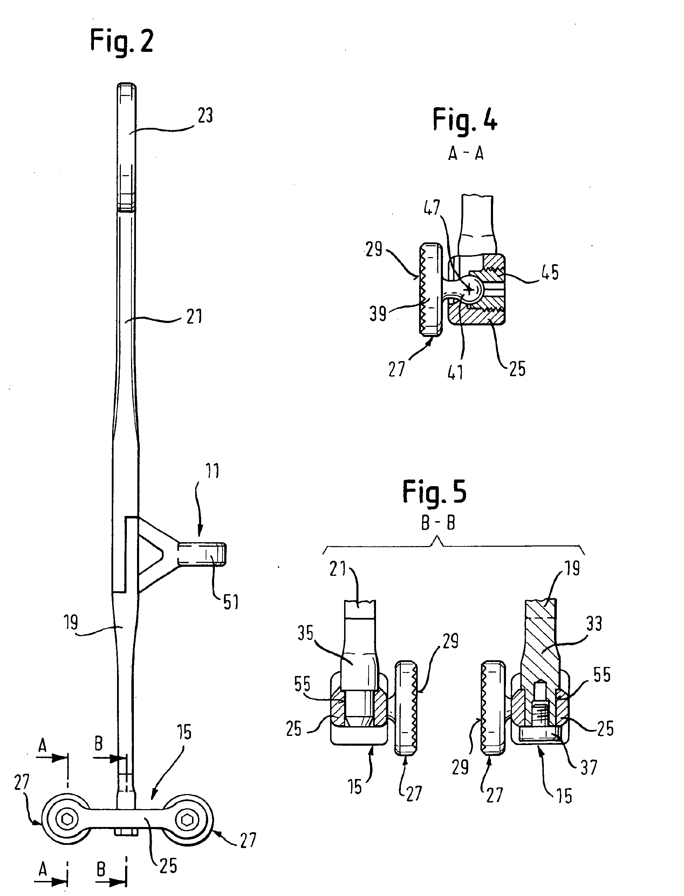

Die Anbringung der beiden Querträger 25 an den freien Enden der Hebel 19, 21 unterscheidet sich dadurch, dass der eine Querträger 25 (in Fig. 5 der linke) fest - z.B. durch Einpressen und/oder Schweissen - mit dem betreffenden Hebel 21 verbunden ist, so dass der Hebel 21 und der Querträger 25 ein starres T bilden, während der andere Querträger 25 (in Fig. 5 der rechte) am betreffenden Hebel 19 drehbar gelagert angebracht ist, wobei die Drehachse mit der Mittelachse des Hebels 19 zusammenfällt und damit senkrecht zur Längserstreckung des Querträgers 25 verläuft.

Die unterschiedliche Anbringung der Querträger 25 an den Hebeln 19, 21 erfolgt mittels unterschiedlich ausgestalteter Anschlusszapfen 33, 35, die an ihrem einen Ende fest mit dem freien Ende des jeweiligen Hebels 19, 21 verbunden sind und auf deren freies Ende der jeweilige Querträger 25 aufgesteckt wird, der hierzu eine mittig angeordnete Bohrung 55 aufweist. Während die Verbindung zwischen dem Querträger 25 und dem Anschlusszapfen 35 des Hebels 21 drehfest ausgestaltet ist, kann der auf dem Anschlusszapfen 33 des Hebels 19 gelagerte Querträger 25 relativ zum Anschlusszapfen 33 gedreht werden. Die Befestigung des Querträgers 25 am Anschlusszapfen 33 erfolgt durch eine Befestigungsschraube 37, die mit dem Anschlusszapfen 33 verschraubt wird.

Die Klemmorgane 27 umfassen jeweils eine zylindrische Klemmplatte 39 mit einer kreisförmigen Klemmfläche 29 sowie einen senkrecht von der der Klemmfläche 29 gegenüber liegenden Seite der Klemmplatte 39 abstehenden Lagerabschnitt 41 mit einem kugelförmigen Lagerkopf.

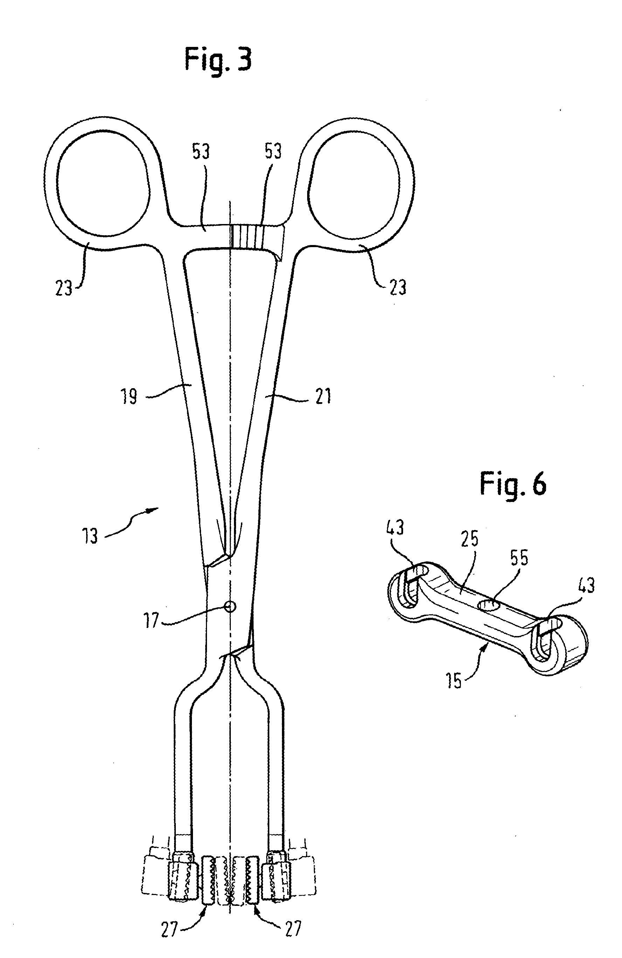

Die Querträger 25 weisen jeweils in ihren erweiterten Endbereichen einen Aufnahmeraum 43 (vgl. insbesondere Fig. 6) für den Lagerabschnitt 41 des jeweiligen Klemmorgans 27 auf. In diesen Aufnahmeraum 43 ist der kugelförmige Lagerkopf (in Fig. 6 von oben) einschiebbar.

Eine Verschlussschraube 45, die von hinten in den Aufnahmeraum 43 einschraubbar ist, dient als Lager für den Lagerabschnitt 41 des Klemmorgans 27. Hierzu ist die Verschlussschraube 45 an ihrer dem Klemmorgan 27 zugewandten Seite als Napflager ausgebildet, das zusammen mit dem Lagerabschnitt 41 ein Kugelgelenk bildet. Hierdurch ist das Klemmorgan 27 um einen bezüglich des Querträgers 25 ortsfesten Schwenkpunkt 47 in alle Richtungen frei bewegbar, wodurch unterschiedliche Orientierungen der Klemmfläche 29 relativ zu dem Querträger 25 möglich sind. Gleichzeitig ist das Klemmorgan 27 durch die Verschlussschraube 45 gegen unerwünschte Translationsbewegungen relativ zum Querträger 25 gesichert.

Die Klemmflächen 29 sind aufgeraut oder - beispielsweise durch Vorsehen einer Mehrzahl von rechtwinklig zueinander angeordneten Kerben - strukturiert, um ein sicheres und ortsstabiles Festklemmen der erfindungsgemäßen Klemmzange 13 an den Rückenwirbeln des Patienten zu gewährleisten.

Die bewegliche Lagerung der Klemmorgane 27 zusammen mit der Verdrehbarkeit des einen Querträgers 25 erlaubt beliebige Relativstellungen der vier Klemmflächen 29 und gewährleistet somit eine optimale Anpassung der Klemmzange 13 an die jeweils zu überbrückenden Rückenwirbel.

In Fig. 3 sind schematisch unterschiedliche Stellungen der die Querträger 25 und die Klemmorgane 27 umfassenden Klemmbacken 15 angedeutet.

Eine zur Koppelung mit einer nicht dargestellten separaten Zielvorrichtung - beispielsweise einer Zielvorrichtung, wie sie im Einleitungsteil beschrieben ist - dienende Zielbasis 11 der erfindungsgemäßen Zielhilfe ist fest mit der Klemmzange 13 verbunden, beispielsweise durch Verschweißen. In dem dargestellten Ausführungsbeispiel steht die Zielbasis 11 senkrecht von dem einen Hebel 19 ab und erstreckt sich somit parallel zu den Klemmbacken 15. Die Zielbasis 11 ist Y-förmig ausgebildet, wobei die beiden Y-Schenkel mit ihren freien Enden mit dem Hebel 19 verschweißt sind und der gerade Y-Abschnitt von einem die eigentliche Basis bildenden Ringabschnitt 51 gebildet ist, über den die Koppelung mit der separaten Zielvorrichtung erfolgt.

Die Ausgestaltung der Zielbasis 11 bzw. des Ringabschnittes 51 ist grundsätzlich beliebig und an die zu koppelnde Zielvorrichtung angepasst.

Um die Zielbasis 11 relativ zu Rückenwirbeln eines Patienten räumlich festzulegen, wird die Klemmzange 13 im geöffneten Zustand mit den Klemmorganen 27 der Klemmbacken 15 an die Dornfortsätze zweier benachbarter Rückenwirbel angesetzt. Der Mittenabstand der Klemmorgane 27 eines jeden Querträgers 25 ist entsprechend dem Mittenabstand der Dornfortsätze des jeweiligen Patienten gewählt und beträgt in dem dargestellten Ausführungsbeispiel 32 mm.

Die Fixierung der Klemmzange 13 an den Rückenwirbeln erfolgt durch Zusammendrücken der beiden Betätigungsabschnitte 23. Nach Art einer Ratsche zusammenwirkende Verriegelungsarme 53 der beiden Hebel 19, 21, die aufgrund ihrer Orientierung relativ zu den Hebeln 19, 21 gegeneinander vorgespannt sind, sichern die jeweils durch Zusammendrücken erreichten Zwischen- und Endstellungen beim Schließen der Klemmzange 13 und verhindern ein Lösen der Klemmung beim Loslassen der Klemmzange 13.

Durch geringfügiges Auseinanderbewegen der beiden Hebel 19, 21 im Bereich der Betätigungsabschnitte 23 senkrecht zu der von ihnen aufgespannten Ebene werden die Verriegelungsarme 53 wieder außer Eingriff gebracht, wodurch die Klemmung wieder gelöst werden kann.

Zur besseren Kontrolle der mit Hilfe der erfindungsgemäßen Zielhilfe bzw. Klemmzange gesetzten Pedikelschrauben mittels Bildverstärker insbesondere in Form von Röntgeneinrichtungen kann die Klemmzange wenigstens zum Teil aus mit Kohlenstofffasern verstärktem Kunststoff hergestellt sein.

### Bezugszeichenliste

- 11: Zielbasis
- 13: Klemmwerkzeug, Klemmzange
- 15: Klemmbacke
- 17: Drehachse
- 19: Hebel
- 21: Hebel
- 23: Betätigungsabschnitt
- 25: Querträger
- 27: Klemmorgan
- 29: Klemmfläche
- 31: Gelenkschraube
- 33: Anschlusszapfen (drehbar)
- 35: Anschlusszapfen (fest)
- 37: Befestigungsschraube
- 39: Klemmplatte
- 41: Lagerabschnitt
- 43: Aufnahmeraum
- 45: Verschlussschraube
- 47: Schwenkpunkt
- 51: Ringabschnitt der Zielbasis
- 53: Verriegelungsarm
- 55: Bohrung

## Patentansprüche

1. Zielhilfe zur räumlichen Festlegung einer Zielvorrichtung relativ zu Rückenwirbeln eines Patienten, mit
einem als Zange ausgebildeten Klemmwerkzeug (13), das wenigstens zwei Klemmbacken (15) aufweist, mit denen das Klemmwerkzeug (13) wenigstens zwei benachbarte Rückenwirbel überbrückend an den Rückenwirbeln festklemmbar ist, und
wenigstens einer fest mit dem Klemmwerkzeug (13) verbundenen Zielbasis (11), die zur Koppelung mit einer separaten Zielvorrichtung ausgebildet ist.

2. Zielhilfe nach Anspruch 1,
**dadurch gekennzeichnet,**
**dass** die Klemmzange (13) zwei um eine Drehachse (17) drehbar miteinander verbundene Hebel (19, 21) aufweist, die jeweils auf der einen Seite der Drehachse (17) mit einem Betätigungsabschnitt (23) versehen sind und auf der anderen Seite der Drehachse (17) eine der Klemmbacken (15) tragen.

3. Zielhilfe nach Anspruch 2,
**dadurch gekennzeichnet,**
**dass** die Hebel (19, 21) zur Sicherung eines hergestellten Klemmzustands relativ zueinander festsetzbar sind.

4. Zielhilfe nach Anspruch 2 oder 3,
**dadurch gekennzeichnet,**
**dass** im festgeklemmten Zustand die Hebel (19, 21) zumindest im Bereich der Klemmbacken (15) im Wesentlichen parallel zueinander verlaufen.

5. Zielhilfe nach einem der Ansprüche 2 bis 4,
**dadurch gekennzeichnet,**
**dass** die Klemmbacken (15) sich im Wesentlichen senkrecht zu einer von den beiden Hebeln (19, 21) aufgespannten Ebene erstrecken.

6. Zielhilfe nach einen der vorhergehenden Ansprüche,
**dadurch gekennzeichnet,**
**dass** die eine Klemmbacke (15) drehbar und die andere Klemmbacke (15) fest an der Klemmzange (13) angebracht ist.

7. Zielhilfe nach einem der Ansprüche 2 bis 6,
**dadurch gekennzeichnet,**
**dass** zumindest eine Klemmbacke (15) einen sich im Wesentlichen senkrecht zu einer von den beiden Hebeln (19, 21) aufgespannten Ebene erstreckenden Querträger (25) umfasst, der wenigstens zwei mit Abstand voneinander angeordnete Klemmorgane (27) trägt, die jeweils eine der anderen Klemmbacke (15) zugewandte Klemmfläche (29) aufweisen.

8. Zielhilfe nach Anspruch 7,
**dadurch gekennzeichnet ,**
**dass** die Klemmflächen (29) jeweils kreisförmig sind.

9. Zielhilfe nach Anspruch 7 oder 8,
**dadurch gekennzeichnet,**
**dass** die Klemmflächen (29) jeweils aufgeraut oder strukturiert sind.

10. Zielhilfe nach einem der Ansprüche 7 bis 9,
**dadurch gekennzeichnet,**
**dass** die Klemmflächen (29) jeweils von einer Mehrzahl von Kerben durchzogen sind.

11. Zielhilfe nach einem der Ansprüche 7 bis 10,
**dadurch gekennzeichnet,**
**dass** die Klemmorgane (27) jeweils am Querträger (25) beweglich gelagert sind.

12. Zielhilfe nach einem der Ansprüche 7 bis 11,
**dadurch gekennzeichnet,**
**dass** zwischen den Klemmorganen (27) und dem Querträger (25) jeweils eine Gelenkverbindung vorgesehen ist, insbesondere eine Kugelgelenkverbindung.

13. Zielhilfe nach einem der Ansprüche 7 bis 12,
**dadurch gekennzeichnet,**
**dass** der Abstand zwischen zwei benachbarten Klemmorganen (27) einer Klemmbacke (15) dem Mittenabstand der Dornfortsätze zweier benachbarter Rückenwirbel im Lendenbereich entspricht.

14. Zielhilfe nach einem der Ansprüche 7 bis 13,
**dadurch gekennzeichnet,**
**dass** der Abstand zwischen zwei benachbarten Klemmorganen (27) einer Klemmbacke (15) im Bereich von 27 mm bis 37 mm liegt.

15. Zielhilfe nach einem der vorhergehenden Ansprüche,
**dadurch gekennzeichnet,**
**dass** die Zielbasis (11) sich etwa parallel zu den Klemmbacken (15) erstreckend von der Klemmzange (13) absteht.

16. Zielhilfe nach einem der vorhergehenden Ansprüche,
**dadurch gekennzeichnet,**
**dass** die Zielbasis (11) etwa senkrecht von einem Hebel (19) der Klemmzange (13) absteht.

17. Zielhilfe nach einem der Ansprüche 2 bis 16,
**dadurch gekennzeichnet,**
**dass** die Zielbasis (11) mit einem der Hebel (19, 21) fest verbunden ist, insbesondere im Bereich der Drehachse (17) der beiden Hebel (19,21).

18. Zielhilfe nach einem der vorhergehenden Ansprüche,
**dadurch gekennzeichnet,**
**dass** sie zumindest teilweise aus mit Kohlenstofffasern verstärktem Kunststoff hergestellt ist.

19. Zielsystem mit einer Zielhilfe nach einem der vorhergehenden Ansprüche und mit wenigstens einer separaten Zielvorrichtung, die mit der Zielbasis (11) der Zielhilfe koppelbar ist.
